# EUROPEAN PATENT SPECIFICATION

(11) **EP 1 409 021 B1**
(45) Date of publication and mention of the grant of the patent: **14.11.2007**
(21) Application number: 02745579.9
(22) Date of filing: 03.07.2002
(51) Int. Cl.: A61K 47/00

(54) **FORMULATION COMPRISING FULVESTRANT**
ZUSAMMENSETZUNG MIT FULVESTRANT
PREPARATION AVEC FULVESTRANT

(30) Priority: 07.07.2001 GB 0116620
(43) Date of publication of application: 21.04.2004
(73) Proprietor: AstraZeneca AB, 151 85 Södertälje (SE)
(72) Inventor: GELLERT, Paul, Richard, Cheshire SK10 4TG (GB); POTTS, Alison, Margaret, Cheshire SK10 4TG (GB)
(86) International application number: PCT/GB2002/003084
(87) International publication number: WO 2003/006063

(56) References cited:
- EP-A- 0 346 014
- WO-A-01/51056
- WO-A-01/74366
- HOWELL, A. ET AL: "Response to a specific antioestrogen (ICI 182780) in tamoxifen-resistant breast cancer ." LANCET, vol. 345, 1995, pages 29-30, XP009004877
- DEFRIEND D.J. ET AL.: "Investigation of a new pure antiestrogen" CANCER RESEARCH, vol. 54, no. 2, 1994, pages 408-414, XP001122348

## Description

The invention relates to a pharmaceutical formulation, preferably adapted for administration by injection , containing the compound 7α-[9-(4,4,5,5,5-pentafluoropentylsulphinyl)nonyl]oestra-1,3,5(10)-triene-3,17β-diol and an antioxidant, more particularly to a formulation adapted for administration by injection containing the compound 7α-[9-(4,4,5,5,5-pentafluoropentylsulphinyl)nonyl]oestra-1,3,5(10)-triene-3,17β-diol in solution in a ricinoleate vehicle which comprises an antioxidant.

Oestrogen deprivation is fundamental to the treatment of many benign and malignant diseases of the breast and reproductive tract. In premenopausal women, this is achieved by the ablation of ovarian function through surgical, radiotherapeutic, or medical means, and, in postmenopausal women, by the use of aromatase inhibitors.

An alternative approach to oestrogen withdrawal is to antagonise oestrogens with antioestrogens. These are drugs that bind to and compete for oestrogen receptors (ER) present in the nuclei of oestrogen-responsive tissue. Conventional nonsteroidal antioestrogens, such as tamoxifen, compete efficiently for ER binding but their effectiveness is often limited by the partial agonism they display, which results in an incomplete blockade of oestrogen-mediated activity (Furr and Jordan, Pharmacology & Therapeutics, 25:127-206, 1984; May and Westley, J Biol Chem 262:15894-15899, 1987).

The potential for nonsteroidal antioestrogens to display agonistic properties prompted the search for novel compounds that would bind ER with high affinity without activating any of the normal transcriptional hormone responses and consequent manifestations of oestrogens. Such molecules would be "pure" antioestrogens, clearly distinguished from tamoxifen-like ligands and capable of eliciting complete ablation of the trophic effects of oestrogens. Such compounds are referred to as Estrogen Receptor-Downregulators (E.R.D.). The rationale for the design and testing of novel, pure antioestrogens has been described in: Bowler et al 1989, Wakeling 1990a, 1990b, 1990c. Wakeling and Bowler 1987, 1988.

Steroidal analogues of oestradiol, with an alkylsulphinyl side chain in the 7α position, provided the first examples of compounds devoid of oestrogenic activity (Bowler et al 1989). One of these, 7α-[9-(4,4,5,5,5-pentafluoropentyl sulphinyl)nonyl]oestra-1,3,5-(10)triene-3,17β-diol was selected for intensive study on the basis of its pure oestrogen antagonist activity and significantly increased antioestrogenic potency over other available antioestrogens. *In vitro* findings and early clinical experience with 7α-[9-(4,4,5,5,5-pentafluoropentylsulphinyl)nonyl]oestra-1,3-5(10)-triene-3,17β-diol have promoted interest in the development of the drug as a therapeutic agent for oestrogen-dependent indications such as breast cancer and certain benign gynaecological conditions.

7α-[9-(4,4,5,5,5-Pentafluoropentylsulphinyl)nonyl]oestra-1,3-5(10)-triene-3,17β-diol, or ICI 182,780, has been allocated the international non-proprietary name fulvestrant, which is used hereinafter. When referring to fulvestrant we include pharmaceutically-acceptable salts thereof and any possible solvates of either thereof.

Fulvestrant binds to ER with an affinity similar to that of oestradiol and completely blocks the growth stimulatory action of oestradiol on human breast cancer cells *in vitro;* it is more potent and more effective than tamoxifen in this respect. Fulvestrant blocks completely the uterotrophic action of oestradiol in rats, mice and monkeys, and also blocks the uterotrophic activity of tamoxifen.

Because fulvestrant has none of the oestrogen-like stimulatory activity that is characteristic of clinically available antioestrogens such as tamoxifen or toremifene, it may offer improved therapeutic activity characterised by more rapid, complete, or longer-lasting tumour regression; a lower incidence or rate of development of resistance to treatment; and a reduction of tumour invasiveness.

European Patent Application No. 0 138 504 discloses that certain steroid derivatives are effective antioestrogenic agents. The disclosure includes information relating to the preparation of the steroid derivatives. In particular there is the disclosure within Example 35 of the compound 7α-[9-(4,4,5,5,5-pentafluoropentylsulphinyl)nonyl]oestra-1,3,5(10)-triene-3,17β-diol, which compound is specifically named in Claim 4. It is also disclosed that the compounds of that invention may be provided for use in the form of a pharmaceutical composition comprising a steroid derivative of the invention together with a pharmaceutically-acceptable diluent or carrier. It is stated therein that the composition can be in a form suitable for oral or parenteral administration.

EP-A-0 346 014 discloses a formulation comprising fulvestrant which can be preserved by the addition of an antioxidant such as ascorbic acid.

Our earlier filed application PCT/GB01/00049, WO 01/51506, describes certain fulvestrant formulations at a most preferred concentration of 50mg/ml. There is a need for formulations of fulvestrant to have greater stability on storage. The present invention is based on the discovery that addition of an antioxidant can improve the stability of fulvestrant formulations.

According to one aspect of the present invention there is provided a pharmaceutical formulation according to claim 1 comprising fulvestrant and an antioxidant selected from:
thiourea;
tocopherol optionally in the presence of lecithin or ascorbyl palmitate;
ascorbyl palmitate;
propyl gallate optionally in the presence of butylated hydroxyanisole or butylated hydroxytoluene;
dithiothreitol;
butylated hydroxytoluene;
dithioerythreitol;
acetyl cysteine;
thioglycerol; and
thioglycolic acid.

Preferably the antioxidant is selected from:
thiourea;
tocopherol optionally in the presence of lecithin or ascorbyl palmitate;
ascorbyl palmitate;
propyl gallate optionally in the presence of butylated hydroxyanisole or butylated hydroxytoluene; and
dithiothreitol.

More preferably the antioxidant is selected from:
thiourea;
tocopherol optionally in the presence of lecithin or ascorbyl palmitate;
propyl gallate optionally in the presence of butylated hydroxyanisole; and
ascorbyl palmitate.

More preferably the antioxidant is selected from:
thiourea; and
tocopherol optionally in the presence of lecithin or ascorbyl palmitate.

More preferably the antioxidant is selected from:
thiourea; and
tocopherol in the presence of lecithin.

Most preferably the antioxidant is thiourea.

The formulation is adapted for intramuscular administration.

The formulation comprises a ricinoleate excipient, a pharmaceutically acceptable non-aqueous ester solvent and a pharmaceutically acceptable alcohol.

Preferably the pharmaceutically-acceptable alcohol is a mixture of ethanol and benzyl alcohol.

Preferably the pharmaceutically-acceptable non-aqueous ester solvent is selected from benzyl benzoate, ethyl oleate, isopropyl myristate, isopropyl palmitate or a mixture of any thereof. More preferably the pharmaceutically-acceptable non-aqueous ester solvent is benzyl benzoate.

According to another aspect of the invention there is provided a unit dose of a pharmaceutical formulation as described herein wherein the total amount of fulvestrant in the formulation is 250mg, or more, and the total volume of the formulation is 6ml, or less.

Preferably the pharmaceutically-acceptable alcohol is a mixture of 10% weight of ethanol per volume of formulation, 10% weight of benzyl alcohol per volume of formulation and 15% weight of benzyl benzoate per volume of formulation and the ricinoleate vehicle is castor oil.

More preferably the pharmaceutical formulation comprises an antioxidant selected from:
0.05 % weight of thiourea per volume of formulation; or
0.075% weight of tocopherol per volume of formulation and 2.3% weight of lecithin per volume of formulation.

Another aspect of the invention provides a pharmaceutical formulation as defined herein for use in medical therapy.

Another aspect of the invention provides use of fulvestrant in the preparation of a pharmaceutical formulation as defined herein for the treatment of a benign or malignant disease of the breast or reproductive tract.

According to one aspect of the present invention there is provided a pharmaceutical formulation adapted for intramuscular administration comprising fulvestrant and an antioxidant selected from ascorbyl palmitate, butylated hydroxyanisole, butylated hydroxytoluene, malic acid, propyl gallate, sodium bisulfite, sodium sulfite, sodium metabisulfite, potassium metabisulfite, potassium bisulfite, sodium thiosulfate, sodium formaldehyde sulfoxylate, L-ascorbic acid, D-ascorbic acid, acetylcysteine, cysteine, thioglycerol, thioglycollic acid, thiolactic acid, thiourea, dithiothreitol, dithioerythreitol, glutathione, nordihydroguaiaretic acid, tocopherol and fumaric acid.

Preferably the antioxidant is selected from ascorbyl palmitate, butylated hydroxyanisole, butylated hydroxytoluene, malic acid, propyl gallate, tocopherol, fumaric acid, sodium ascorbate, sodium metabisulfite and ascorbic acid. More preferably the antioxidant is selected from ascorbyl palmitate, butylated hydroxyanisole, butylated hydroxytoluene, malic acid, propyl gallate, tocopherol and fumaric acid. More preferably the antioxidant is selected from ascorbyl palmitate, butylated hydroxyanisole, butylated hydroxytoluene, malic acid and propyl gallate. More preferably the antioxidant is selected from ascorbyl palmitate, butylated hydroxyanisole, butylated hydroxytoluene and propyl gallate. More preferably the antioxidant is selected from ascorbyl palmitate, butylated hydroxyanisole and propyl gallate. More preferably the antioxidant is selected from butylated hydroxyanisole and propyl gallate. Most preferably, the antioxidant is butylated hydroxyanisole especially at a concentration of about 0.03 %.

Preferably a pharmaceutical formulation of the invention comprises a ricinoleate excipient, a pharmaceutically acceptable non-aqueous ester solvent and a pharmaceutically acceptable alcohol. Preferably the pharmaceutically-acceptable alcohol is a mixture of ethanol and benzyl alcohol. Preferably the pharmaceutically-acceptable non-aqueous ester solvent is selected from benzyl benzoate, ethyl oleate, isopropyl myristate, isopropyl palmitate or a mixture of any thereof. More preferably the pharmaceutically-acceptable non-aqueous ester solvent is benzyl benzoate.

According to another aspect of the present invention there is provided a unit dose of a pharmaceutical formulation as described herein wherein the total amount of fulvestrant in the formulation is 250mg, or more, and the total volume of the formulation is 6ml, or less. 15. A preferred pharmaceutical formulation is one wherein the pharmaceutically-acceptable alcohol is a mixture of 10% weight of ethanol per volume of formulation, 10% weight of benzyl alcohol per volume of formulation and 15% weight of benzyl benzoate per volume of formulation and the ricinoleate vehicle is castor oil.

Another aspect of the invention provides a pharmaceutical formulation adapted for intramuscular injection as defined herein for use in medical therapy.

Another aspect of the invention provides use of fulvestrant in the preparation of a pharmaceutical formulation as defined in any preceding claim for the treatment of a benign or malignant disease of the breast or reproductive tract.

For the avoidance of any doubt when using the term % weight per volume of formulation for the constituents of the formulation we mean that within a unit volume of the formulation a certain percentage of the constituent by weight will be present, for example a 1% weight per volume formulation will contain within a 100ml volume of formulation 1g of the constituent. By way of further illustration

| % of x by weight per volume of formulation | weight of x in 1 ml of formulation |
|---|---|
| 30% | 300mg |
| 20% | 200mg |
| 10% | 100mg |
| 5% | 50mg |
| 1% | 10mg |

It is appreciated that in the formulation an excess of formulation may be included to allow the attendant physician or care giver to be able to deliver the required dose. Therefore, when a 5ml dose is required it would be appreciated that an excess of up to 0.25ml, preferably up to 0.15ml will also be present in the formulation. Typically the formulation will be presented in a vial or a prefilled syringe, preferably a prefilled syringe, containing a unit dosage of the formulation as described herein, these being further features of the invention.

It will be understood by the skilled person that the pharmaceutically-acceptable alcohol will be of a quality such that it will meet pharmacopoeial standards (such as are described in the US, British, European and Japanese pharmacopoeias) and as such will contain some water and possibly other organic solvents, for example ethanol in the US Pharmacopeia contains not less than 94.9% by volume and not more than 96.0% by volume of ethanol when measured at 15.56°C. Dehydrated alcohol in the US Pharmacopeia contains not less than 99.5% ethanol by volume when measured at 15.56°C.

It will be understood by the skilled person that the pharmaceutically-acceptable non-aqueous ester solvent will be of a quality that it will meet pharmacopoeial standards (such as described in the US, British, European and Japanese pharmacopoeias).

Additional excipients commonly used in the formulation field including, for example, a colorant or a surfactant may be used. A preferred optional excipient is a surfactant.

Another aspect of the invention is the addition of a synergistic compound to the formulations described herein to further improve the performance thereof. Examples of synergistic compounds are set out below.

### Combinations of antioxidants which demonstrate Antioxidant Synergy

| **Antioxidant** | **Synergistic Compound** |
|---|---|
| Acetylcysteine | Ascorbic Acid |
| Tocopherol | Lecithin |
| Tocopherol | Ascorbyl Palmitate |
| Butylated Hydroxyanisole | Citric Acid |
| Butylated Hydroxytoluene | Malic Acid |
| Propyl Gallate | Butylated Hydroxyanisole |
| Propyl Gallate | Butylated Hydroxytoluene |

### Other compounds which are Antioxidant Synergists

Ethylenediametetraacetic Acid (EDTA) + its sodium and calcium salts
Citric Acid
Phosphoric Acid
Tartaric Acid
PVP
Lecithin
Glycerin
Propylene Glycol
Polyethylene Glycol
Polysorbates
Glycine
Cysteine
Tryptophan

The synergist can be an antioxidant or a compound which in itself does not protect oxygen sensitive drugs but when combined with an antioxidant, increases the stability of the drug to oxidation.

In addition to fulvestrant another similar type of molecule is currently under clinical investigation. SH-646 (11β-fluoro-7α-(14,14,15,15,15-pentafluoro-6-methyl-10-thia-6-azapentadecyl)estra-1,3,5(10)-triene-3,17β-diol) is also putatively a compound with the same mode of action as fulvestrant and has a very similar chemical structure. It is believed that the compound will also share with fulvestrant similar physical properties and therefore the current invention will also have application with this compound.

Further features of the invention are those as described above but in which SH-646 is substituted for fulvestrant.

### References

1. Bowler J, Lilley TJ, Pittam JD, Wakeling AE. Novel steroidal pure antioestrogens. Steroids, 54: 71-100, 1989.
2. Wakeling AE. Novel pure antioestrogens: mode of action and therapeutic prospects. American New York Academy Science 1990a; 595: 348-56.
3. Wakeling AE. Steroidal pure antioestrogens. In Lippman M, Dickson R, editors. Regulatory mechanisms in breast cancer. Boston: Kluwer Academic, 1990b: 239-57.
4. Wakeling AE. Therapeutic potential of pure antioestrogens in the treatment of breast cancer. Journal Steroid Biochemistry 1990c; 37: 771-5.
5. Wakeling AE, Bowler J. Steroidal pure antioestrogens. Journal Endocrinology 1987; 112: R7-10.
6. Wakeling AE, Bowler J. Biology and mode of action of pure antioestrogens. Journal Steroid Biochemistry 1988; 3: 141-7.

The invention is illustrated below with respect to the following non-limiting Examples.

### Example 1

### Effect of antioxidants on stability

To analyse the following samples of fulvestrant formulations for levels of fulvestrant sulphone by HPLC. The formulations have been stored at 80°C and contain antioxidants which are intended to suppress formation of fulvestrant sulphone. To assess the effect of the antioxidants on formation of fulvestrant sulphone the results will be compared with a reference sample containing no antioxidant and stored at 80°C. The following basic formulation was used: fulvestrant 50mg/ml, ethanol 10% w/v, benzylalcohol 10% w/v, benzyl benzoate 15% w/v and made to volume with castor oil.

**Table 1: Sample Details.**

| **ADM** | **Antioxidant and Storage Conditions** |
|---|---|
| 82843D01 | No antioxidant, 80°C, no nitrogen |
| 82844A01 | No antioxidant, 80°C, no nitrogen |
| 82845101 | No antioxidant, 4°C, under nitrogen |
| 82846F01 | No antioxidant, 4°C, under nitrogen |
| 82848K01 | 0.02 % Ascorbyl Palmitate, 80°C, no nitrogen |
| 82849H01 | 0.02 % Ascorbyl Palmitate, 80°C, no nitrogen |
| 82851F01 | 0.03 % Butylated Hydroxyanisole, 80°C, no nitrogen |
| 82852C01 | 0.03 % Butylated Hydroxyanisole, 80°C, no nitrogen |
| 82854H01 | 0.03 % Butylated Hydroxytoluene, 80°C, no nitrogen |
| 82855E01 | 0.03 % Butylated Hydroxytoluene, 80°C, no nitrogen |
| 82857J01 | 0.02 % Malic Acid, 80°C, no nitrogen |
| 82858G01 | 0.02 % Malic Acid, 80°C, no nitrogen |
| 82860E01 | 0.01 % Propyl Gallate, 80°C, no nitrogen |
| 82861B01 | 0.01 % Propyl Gallate, 80°C, no nitrogen |

### Results

A single sample preparation and single injection was performed on each of the above samples. Due to leakage of the vials at the high storage temperature the fulvestrant content of the samples could not be assumed to be the same in each vial. Therefore, the level of fulvestrant sulphone in each sample was calculated using an external fulvestrant reference standard and also relative to the fulvestrant content of the sample itself. Both sets of results are shown below.

**Table 2: Fulvestrant Sulphone content (% w/w) determined against an external fulvestrant analytical reference standard.**

| **Formulation and Storage Conditions** | **ADM** | **Fulvestrant sulphone (% w/w)** | **Fulvestrant sulphone (mean % w/w)** |
|---|---|---|---|
| No antioxidant, 80°C, no nitrogen | 1. 82843D01 | 4.779 | 4.94 |
| | 2. 82844A01 | 5.092 | |
| No antioxidant, 4°C, under nitrogen | 1. 82845101 | 0.202 | 0.20 |
| | 2. 82846F01 | 0.188 | |
| 0.02 % Ascorbyl Palmitate, 80°C, no nitrogen | 1. 82848K01 | 3.530 | 3.28 |
| | 2. 82849H01 | 3.035 | |
| 0.03 % Butylated Hydroxyanisole, 80°C, no nitrogen | 1. 82851F01 | 1.756 | 2.28 |
| | 2. 82852C01 | 2.794 | |
| 0.03 % Butylated Hydroxytoluene, 80°C, no nitrogen | 1. 82854H01 | 3.424 | 3.53 |
| | 2. 82855E01 | 3.628 | |
| 0.02 % Malic Acid, 80°C, no nitrogen | 1. 82857J01 | 4.129 | 4.00 |
| | 2. 82858G01 | 3.880 | |
| 0.01 % Propyl Gallate, 80°C, no nitrogen | 1. 82860E01 | 3.040 | 3.33 |
| | 82861B01 | 3.620 | |

**Table 3: Fulvestrant Sulphone content (area %) relative to the fulvestrant content of the sample.**

| **Formulation and Storage Conditions** | **ADM** | **Fulvestrant Sulphone (area %)** | **Fulvestrant Sulphone (mean area %)** |
|---|---|---|---|
| No antioxidant, 80°C, no nitrogen | 1. 82843D01 | 4.020 | 4.03 |
| | 2. 82844A01 | 4.035 | |
| No antioxidant, 4°C, under nitrogen | 1. 82845I01 | 0.187 | 0.18 |
| | 2. 82846F01 | 0.176 | |
| 0.02 % Ascorbyl Palmitate, 80°C, no nitrogen | 1. 82848K01 | 2.750 | 2.70 |
| | 2. 82849H01 | 2.644 | |
| 0.03 % Butylated Hydroxyanisole, 80°C, no nitrogen | 1. 82851F01 | 1.435 | 1.79 |
| | 2. 82852C01 | 2.145 | |
| 0.03 % Butylated Hydroxytoluene, 80°C, no nitrogen | 1.82854H01 | 2.996 | 2.78 |
| | 2. 82855E01 | 2.561 | |
| 0.02 % Malic Acid, 80°C, no nitrogen | 1. 82857J01 | 3.658 | 3.29 |
| | 2. 82858G01 | 2.196 | |
| 0.01 % Propyl Gallate, 80°C, no nitrogen | 1. 82860E01 | 2.136 | 2.40 |
| | 2. 82861B01 | 2.661 | |

### Conclusion

The results indicate that the addition of an antioxidant to the fulvestrant formulation reduces formation of fulvestrant sulphone by between 1-2 % w/w, when compared to a formulation stored under the same conditions in the absence of antioxidant. The lowest level of fulvestrant sulphone was observed with the use of butylated hydroxyanisole. None of the antioxidants co-eluted with the fulvestrant or the fulvestrant sulphone peaks.

### Example 2

### Formulation

Fulvestrant is mixed with alcohol and benzyl alcohol, stirring until completely dissolved. Benzyl benzoate is added and the solution is made to final weight with castor oil and stirred, (for convenience weight is used rather than volume by using the weight to volume ratio). The bulk solution is overlaid with nitrogen. The solution is sterilised by filtration using one or two filters of 0.2µm porosity. The sterile filtrate is kept under a nitrogen overlay as it is filled under aseptic conditions into washed and depyrogenised, sterile primary containers, for example vials or pre-filled syringes. An overage is included in the primary pack to facilitate removal of the dose volume. The primary packs are overlaid with sterile nitrogen, before aseptically sealing.

### See also process flow diagram below

Quantities of each component of the formulation is chosen according to the required formulation specification, examples are described above. For example quantities are added of each component to prepare a formulation which contains
10% weight per volume of benzyl alcohol
10% weight per volume of ethanol
15% weight per volume of benzyl benzoate
0.03 % weight per volume of butylated hydroxyanisole (added at same time as alcohols in the flow diagram)
250mg of fulvestrant for each 5ml of finished formulation
and the remaining amount as castor oil.

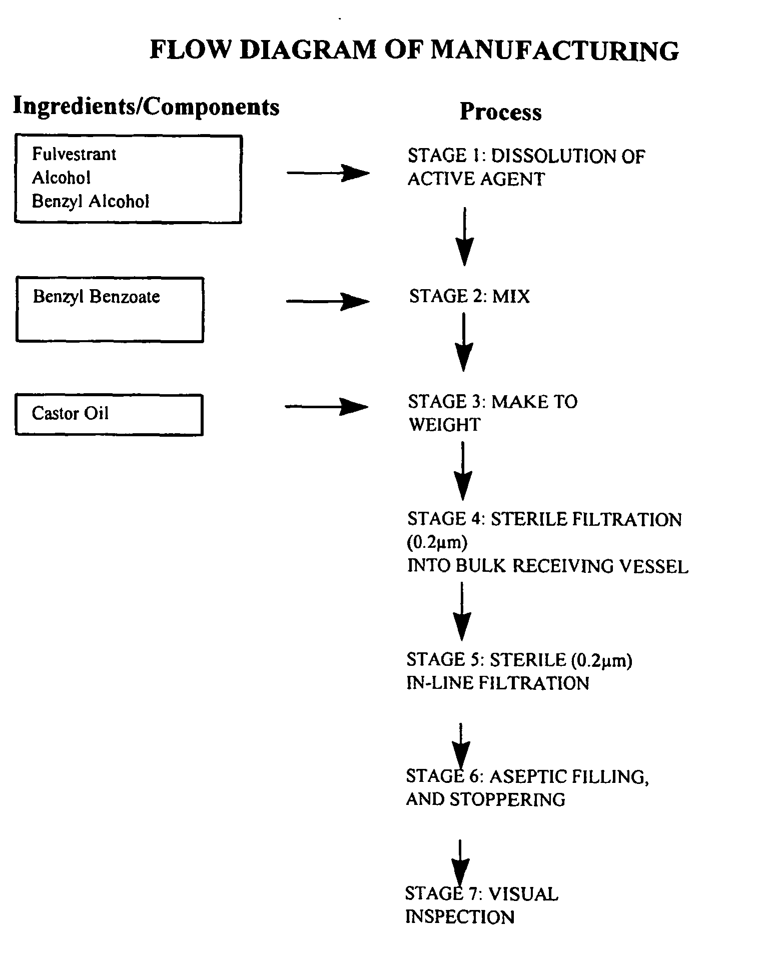

### Example 3

### Stability Study at 40°C/ 75% Relative Humidity with Antioxidants

### 3.1 Introduction

Antioxidants are intended to suppress the formation of oxidative degradation products. This Example describes a stability study to investigate the level of degradation products, particularly Fulvestrant Sulphone (the main degradation product of fulvestrant and is formed by oxidation) formed with and without an antioxidant. The formulations were stored at 40oC/ 75% RH and analysed at 1 month. A range of pharmaceutically acceptable antioxidants are used as well as antioxidants with synergists. "RH" means relative humidity and the percentage relative humidity may be defined as:
(Vapour pressure of water vapour in the air / Vapour pressure of water vapour in air saturated at the same temperature) * 100; see Pharmaceutics The Science of Dosage Form Design., Aulton, M.E. (Ed), Churchill Livingstone, 2002.

The 80°C accelerated stability study described in Example 1 may not be completely indicative of actual/ realistic storage conditions. Although storing the formulations at 40°C/ 75%RH provides an accelerated study, the conditions are more realistic and thought to give a more accurate indication of the ability of an antioxidant to prevent oxidation of fulvestrant. Storage at 40°C/75%RH was therefore employed in this study.

To assess the effect of the antioxidant on the formation of Fulvestrant Sulphone, the results were compared with a control sample containing no antioxidant and stored under the same conditions, with or without nitrogen overlay. The following basic formulation was used: Fulvestrant 50mg/ml, Ethanol 10% w/v, Benzyl alcohol 10% w/v, Benzyl benzoate 15% w/v and made up to volume with castor oil.

All samples used in the study were filled into 5 ml vials with a fill volume of 2.5ml to maximise headspace. The purpose of the study is:
i) To determine the level of Fulvestrant Sulphone and other degradation products in each formulation
ii) To assess the effectiveness of the antioxidant in suppressing the formation of Fulvestrant Sulphone and other degradation products, and thus the degradation of Fulvestrant.

### 3.2 Materials

| Ingredients | % w/v |
|---|---|
| Fulvestrant | 5.0 |
| Ethanol 96% BP | 10.0 |
| Benzyl alcohol PhEur | 10.0 |
| Benzyl benzoate PhEur | 15.0 |
| Antioxidant | variable |
| Castor Oil PhEur | To 100% |

### List of Formulations containing antioxidants

| Formulation Code | Antioxidant | % conc | Synergist | % conc |
|---|---|---|---|---|
| A1 | Ascorbyl palmitate | 0.02 | | |
| A2 | Butylated Hydroxyanisole | 0.03 | | |
| A3 | Butylated Hydroxytoluene | 0.03 | | |
| A4 | Malic Acid | 0.02 | | |
| A5 | Propyl Gallate | 0.1 | | |
| A7 | Acetylcysteine | 0.5 | | |
| A9 | Thioglycerol | 1.0 | | |
| A10 | Thioglycolic Acid | 0.5 | | |
| A11 | Thiourea | 0.05 | | |
| A 12 | Dithiothreitol | 0.1 | | |
| A 13 | Dithioerythreitol | 0.1 | | |
| A14 | Delta Tocopherol | 0.075 | | |
| A 16 | Delta Tocopherol | 0.075 | Lecithin | 2.3 |
| A17 | Delta Tocopherol | 0.075 | Ascorbyl Palmitate | 0.02 |
| A18 | Butylated Hydroxyanisole | 0.03 | Citric Acid | 2.0 |
| A19 | Butylated Hydroxytoluene | 0.03 | Malic Acid | 0.02 |
| A20 | Propyl Gallate | 0.1 | Butylated | 0.03 |
| | | | Hydroxyanisole | |
| A21 | Propyl Gallate | 0.1 | Butylated Hydroxytoluene | 0.03 |
| A23 | (CONTROL with nitrogen overlay) | | | |
| A24 | (CONTROL) | | | |

### 3.4 Method of Manufacture

Manufacture 50ml of each proposed formulation as follows:
1. Dissolve antioxidant & Fulvestrant in Ethanol
2. Add Benzyl Alcohol
3. Add Benzyl Benzoate and mix
4. Add Castor Oil to volume and mix
5. Pre-purge vials with Nitrogen, Fill solution into 5ml vials (Fill volume 2.5mls), overlay with Nitrogen.*
6. Seal with ETFE coated stoppers
7. Crimp and label vials.
* - Nitrogen overlay with control sample A23 only.

### 3.5 Storage Protocol

All formulations (A1 - A24) listed above were placed on test at 40°C/75%RH. Testing was performed on all formulations initially and after 1 month of storage at 40°C/75%RH. All vials were stored upright.

### 3.6 Testing Protocol

The levels of degradation products (Fulvestrant Sulphone, unknown degradation products and total degradation products) were determined by HPLC for the initial and 1 month time-point using the following reversed-phase HPLC method.

### Equipment

- Apparatus :: A suitable liquid chromatograph equipped with a column heater, autosampler/injector, UV detector and a pump with gradient capability
- Column :: 15 cm x 4.6 mm internal diameter packed with 3.5 µm USP packing type L7, such as Symmetry C8 or equivalent

### Chromatographic conditions

| | | | |
|---|---|---|---|
| Eluent A : | Methanol | | 270 ml |
| | Acetonitrile | | 320 ml |
| Eluent B : | Methanol | | 410 ml |
| | Acetonitrile | | 490 ml |

| Gradient programme : | Time (min) | Eluent A (%) | Eluent B (%) |
|---|---|---|---|
| | 0 | 100 | 0 |
| | 25 | 100 | 0 |
| | 55 | 0 | 100 |
| | 65 | 0 | 100 |
| Flow rate : | Approximately 2 ml/min | | |
| Monitoring wavelength : | 225 nm | | |
| Injection volume : | 10 µl | | |
| Column temperature : | 40°C | | |
| Data collection time1 : | 55 min | | |

| | | | |
|---|---|---|---|
| ¹Data collection is stopped at 55 minutes as significant baseline disturbance occurs after this time due to the gradient. | | | |

### Retention data

The relative retention times of fulvestrant and fulvestrant sulphone are given below.

**Table - Relative retention times**

| Component | Approximate retention time (minutes) | Approximate relative retention time (RRT) |
|---|---|---|
| Fulvestrant | 21 | - |
| Fulvestrant Sulphone | 25 | 1.21 |

### 3.7 Results

| **CONDITION** | **40 C/ 75% RH** | | | | | | **Initial & 4 week time point** | | | |
|---|---|---|---|---|---|---|---|---|---|---|
| **Sample** | **A1** | | **A2** | | **A4** | | **A7** | | **A9** | |
| **Degradation Products** | **Initial** | **4 week** | **Initial** | **4 week** | **Initial** | **4 week** | **Initial** | **4 week** | **Initial** | **4 week** |
| Fulvestrant Sulphone | **0.09** | **0.25** | 0.08 | 0.39 | **0.11** | **0.37** | 0.07 | 0.08 | 0.05 | 0.05 |
| Highest Other | **0.05** | **0.08** | 0.05 | 0.24 | **0.05** | **<0.05** | 0.18 | 1.21 | 0.12 | 2.93 |
| Total Deg Products | **0.19 (3)** | **0.33 (2)** | 0.18 (3) | 1.02 (7) | **0.21 (3)** | **0.37** | 0.43 (5) | 1.54 (5) | 0.27 (4) | 3.06 (3) |
| | | | | | | | | | | |
| Visual | **NT** | **OK** | NT | OK | **NT** | **OK** | NT | OK | NT | OK |
| | | | | | | | | | | |

| **Sample** | **A10** | | **A11** | | **A12** | | **A13** | | **A14** | |
|---|---|---|---|---|---|---|---|---|---|---|
| **Degradation Products** | **Initial** | **4 week** | **Initial** | **4 week** | **Initial** | **4 week** | **Initial** | **4 week** | **Initial** | **4 week** |
| Fulvestrant Sulphone | 0.06 | **0.09** | **0.05** | <0.05 | **0.07** | 0.08 | 0.06 | **0.07** | **0.08** | **0.2** |
| Highest Other | 0.38 | 4.22 | **0.05** | **0.05** | **0.05** | 0.3 | 0.05 | 0.47 | **0.05** | **<0.05** |
| Total Deg Products | 0.73 (6) | 4.65 (7) | **0.15 (3)** | **0.05** | 0.17 (3) | **0.38 (2)** | 0.16 (3) | 0.54 (2) | **0.17 (3)** | **0.2** |
| | | | | | | | | | | |
| Visual | NT | **OK** | **NT** | **OK** | NT | OK | NT | OK | **NT** | **OK** |

| **Highest other = highest degradation product other than sulphone** | | | | | | | | | | |
|---|---|---|---|---|---|---|---|---|---|---|
| **CONDITION** | **40 C/ 75% RH** | | | | | | **Initial & 4 week time point** | | | |
| **Sample** | **A3** | | **A5** | | **A16** | | **A17** | | **A18** | |
| **Degradation Products** | **Initial** | **4 week** | **Initial** | **4 week** | **Initial** | **4 week** | **Initial** | **4 week** | **Initial** | **4 week** |
| Fulvestrant Sulphone | 0.11 | 0.18 | 0.06 | 0.11 | **0.07** | **0.17** | **0.1** | **0.26** | 0.09 | 0.75 |
| Highest Other | 0.11 | 0.19 | 0.09 | <0.05 | **0.05** | **<0.05** | **0.06** | **<0.05** | 0.05 | 0.5 |
| Total Deg Products | 0.32 (4) | 0.5 * | 0.20 (3) | 0.11 | **0.22 (4)** | **0.17** | **0.21 (3)** | **0.26** | 0.14 (2) | 3.52 (16) |
| | | | | | | | | | | |
| Visual | NT | OK | NT | Fails | **NT** | **OK** | **NT** | **OK** | NT | Fails |
| | | | | | | | | | | |

| **Sample** | **A19** | | **A20** | | **A21** | | **A23 CONTROL (N2)** | | **A24 CONTROL** | |
|---|---|---|---|---|---|---|---|---|---|---|
| **Degradation Products** | **Initial** | **4 week** | **Initial** | **4 week** | **Initial** | **4 week** | **Initial** | **4 week** | **Initial** | **4 week** |
| Fulvestrant Sulphone | 0.14 | 0.35 | 0.06 | 0.11 | 0.1 | 0.15 | 0.08 | 0.36 | 0.08 | 0.34 |
| Highest Other | 0.05 | 0.06 | 0.05 | 0 | 0.06 | 0.1 | 0.05 | 0 | 0.05 | 0 |
| Total Deg Products | 0.29 (4) | 0.47 (3) | 0.16 (3) | 0.11 | 0.26 (4) | 0.31 (3) | 0.18 (3) | 0.36 | 0.18 | 0.34 |
| Visual | NT | OK | NT | Fails | NT | Fails | NT | OK | NT | OK |

| | | | | | | | | | | |
|---|---|---|---|---|---|---|---|---|---|---|
| * RRT 1.61 of 0.35 %w/w (Beta-isomer retention time) not included in total deg. Products () = number of degradation products in sample NT = not tested RH = relative humidity | | | | | | | | | | |

### 3.8 Analysis - Ranking by Sulphone concentration

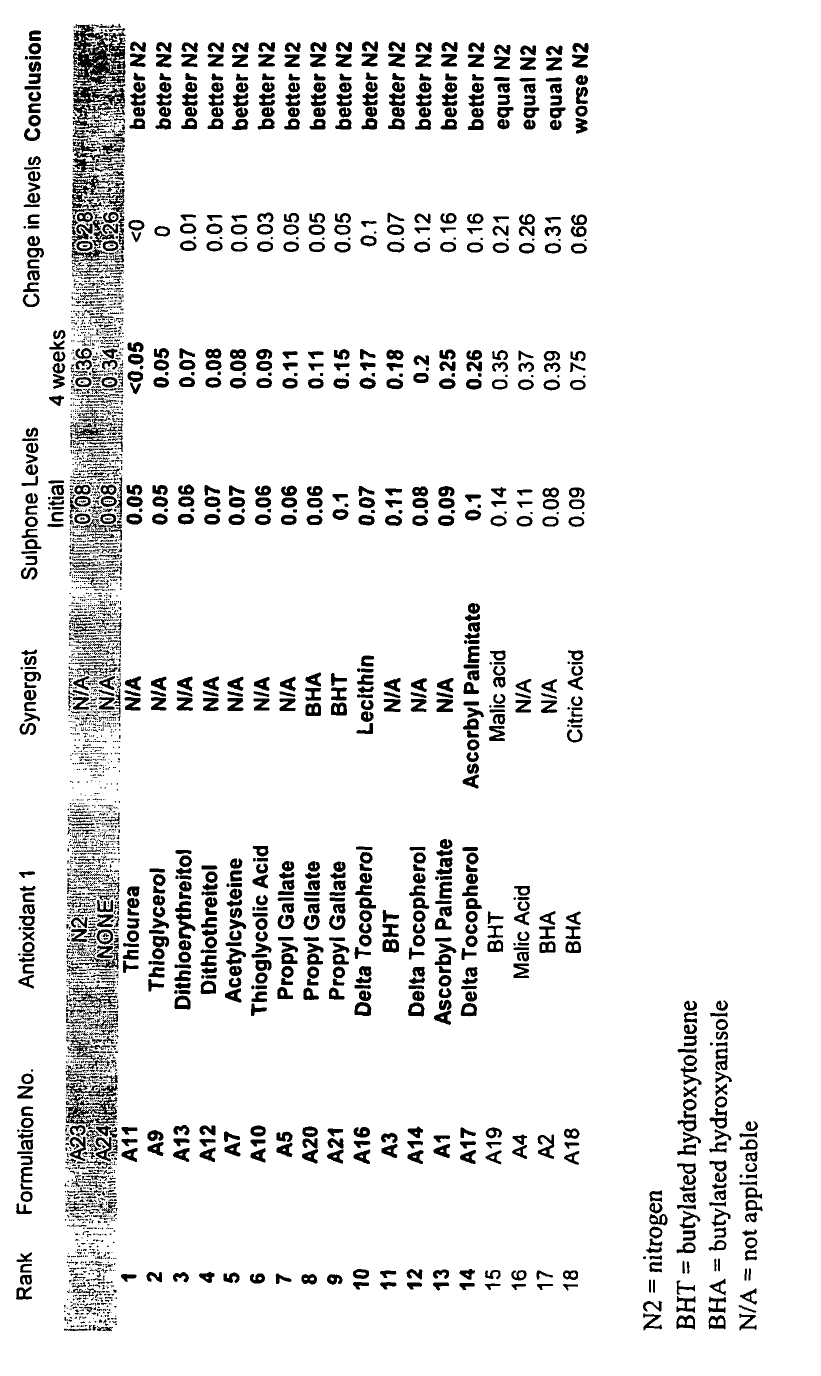

### 3.9 Analysis - Ranking by Total Degradation Products

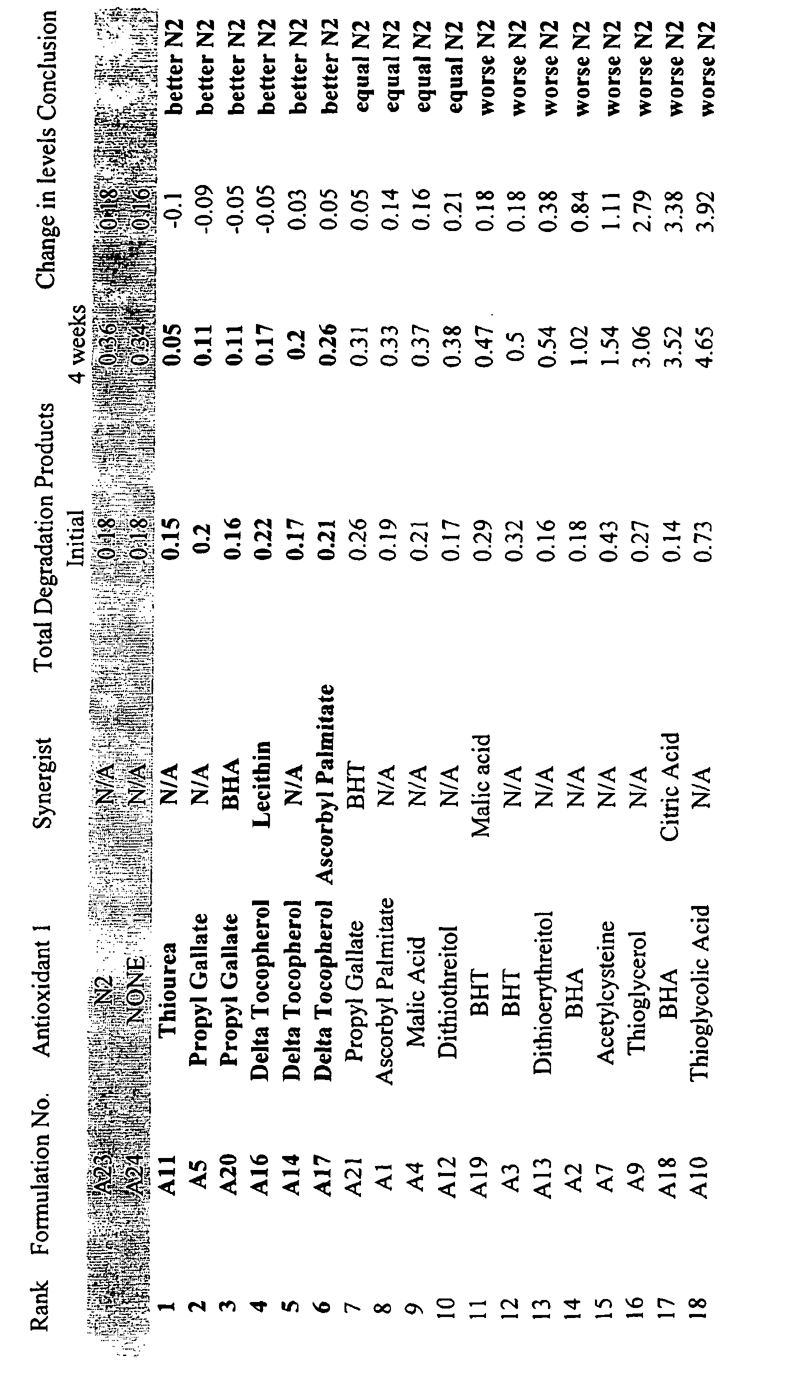

### 3.10 Analysis - Formulations with Sulphone levels < Control and Total Degradation Products < or Equal to Control

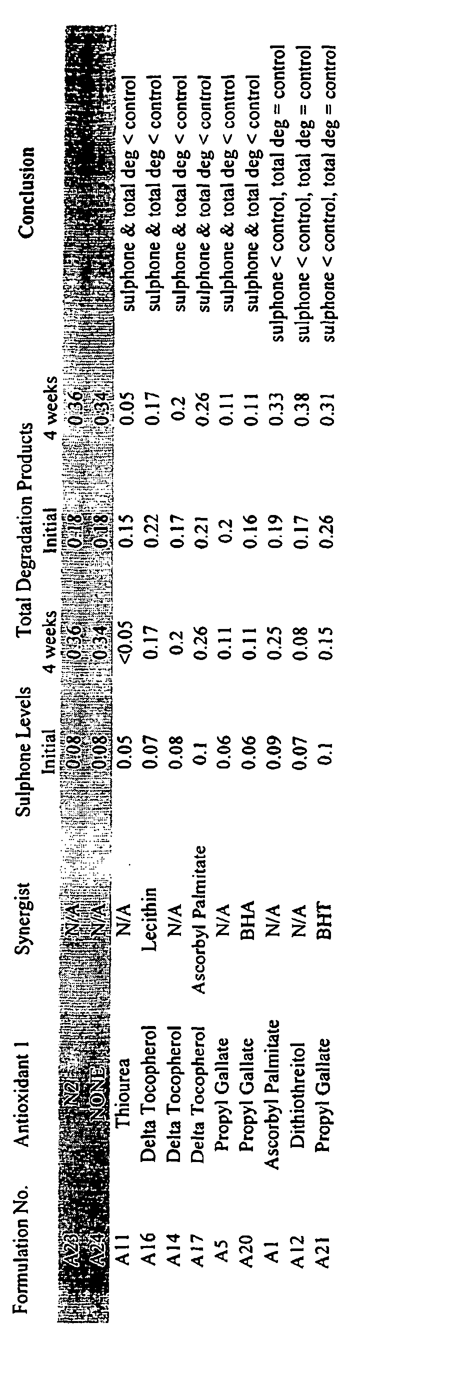

### 3.11 Analysis - Ranking by 'Highest Other' Degradation Product

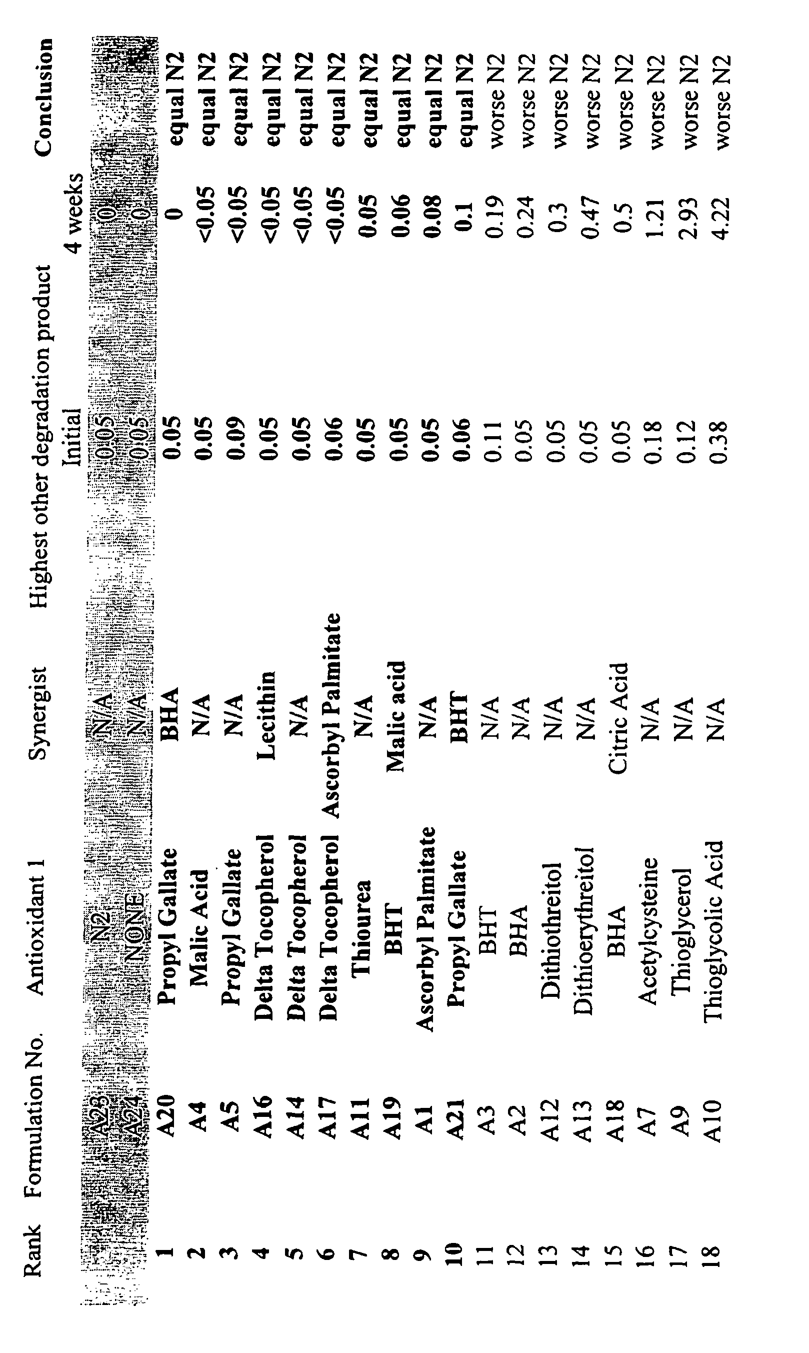

### 3.12 Overall Analysis

| Formulation | Sulphone | Highest Other | Total | Visual |
|---|---|---|---|---|
| A1 | ++ | + | + | + |
| A2 | + | - | - | + |
| A3 | ++ | - | - | + |
| A4 | + | + | + | + |
| A5 | ++ | + | ++ | - |
| A7 | ++ | - | - | + |
| A9 | ++ | - | - | + |
| A10 | ++ | - | - | + |
| A11 | ++ | + | ++ | + |
| A12 | ++ | - | + | + |
| A13 | ++ | - | - | + |
| A14 | ++ | + | ++ | + |
| A16 | ++ | + | ++ | + |
| A17 | ++ | + | ++ | + |
| A18 | - | - | - | - |
| A19 | + | + | - | + |
| A20 | ++ | + | ++ | - |
| A21 | ++ | + | + | - |

| | | | | |
|---|---|---|---|---|
| Code for: ++ = better than control; - = worse than control; + =approximately same as control | | | | |

Taking into account the summary above and the numerical results together, we conclude with the following rank order the best 14 formulations (out of the 18 tested), the best being listed first:
A11>A16>A14>A17>A1 > A5, A20 > A21 > A12 > A3 > A13 > A7 > A9 > A10

### Example 4

### Optimisation of the antioxidant content

Optimisation of the antioxidant content can be conducted as follows. Formulations containing a range of antioxidant concentrations below the level previously shown to be effective (e.g. 0.01-0.05 for thiourea) are placed on stability at 4°C, 25°C and 40°C/ 75%RH for 3 months. The levels of fulvestrant sulphone, total degradation products and highest other degradation products are determined in the initial samples, and after 1 and 3 months of storage. Comparison of the levels of degradation products determines the minimum effective level of antioixidant required. Note excipent concentrations should preferably be minimised for parenteral formulations.

For formulations containing higher fulvestrant content (for example, 150mg/ml) the optimum level of antioxidant as determined previously may be added to the formulation. The formulation is placed on stability study at 4°C, 25°C and 40°C / 75% RH for 3 months and the levels of fulvestrant sulphone, total degradation products and highest other degradation products determined. Should this level of antioxidant be sub-optimal to control oxidation of the fulvestrant, increased levels of the antioxidant may be tested up to the maximum level of antioxidant deemed to be appropriate for incorporation into the product.

## Claims

1. A pharmaceutical formulation adapted for intramuscular administration comprising fulvestrant, a ricinoleate excipient, a pharmaceutically acceptable non-aqueous ester solvent, a pharmaceutically-acceptable alcohol and an antioxidant selected from:
thiourea;
tocopherol optionally in the presence of lecithin or ascorbyl palmitate;
ascorbyl palmitate;
propyl gallate optionally in the presence of butylated hydroxyanisole or butylated hydroxytoluene;
dithiothreitol;
butylated hydroxytoluene;
dithioerythreitol;
acetyl cysteine;
thioglycerol; and
thiolactic acid.

2. A formulation according to claim 1 wherein the antioxidant is selected from:
thiourea;
tocopherol optionally in the presence of lecithin or ascorbyl palmitate;
ascorbyl palmitate;
propyl gallate optionally in the presence of butylated hydroxyanisole or butylated hydroxytoluene; and
dithiothreitol.

3. A formulation according to claim 1 wherein the antioxidant is selected from:
thiourea;
tocopherol optionally in the presence of lecithin or ascorbyl palmitate;
propyl gallate optionally in the presence of butylated hydroxyanisole; and
ascorbyl palmitate.

4. A formulation according to claim 1 wherein the antioxidant is selected from:
thiourea; and
tocopherol optionally in the presence of lecithin or ascorbyl palmitate.

5. A formulation according to claim 1 wherein the antioxidant is selected from:
thiourea; and
tocopherol in the presence of lecithin.

6. A formulation according to claim 1 wherein the antioxidant is thiourea.

7. A pharmaceutical formulation as claimed in any preceding claim wherein the pharmaceutically-acceptable alcohol is a mixture of ethanol and benzyl alcohol.

8. A pharmaceutical formulation as claimed in any preceding claim wherein the pharmaceutically-acceptable non-aqueous ester solvent is selected from benzyl benzoate, ethyl oleate, isopropyl myristate, isopropyl palmitate or a mixture of any thereof.

9. A pharmaceutical formulation as claimed in claim 8 wherein the pharmaceutically-acceptable non-aqueous ester solvent is benzyl benzoate.

10. A unit dose of a pharmaceutical formulation as claimed in any claim from 1 to 9 wherein the total amount of fulvestrant in the formulation is 250mg, or more, and the total volume of the formulation is 6ml, or less.

11. A pharmaceutical forumulation as claimed in any preceding claim wherein the pharmaceutically-acceptable alcohol is a mixture of 10% weight of ethanol per volume of formulation, 10% weight of benzyl alcohol per volume of formulation and 15% weight of benzyl benzoate per volume of formulation and the ricinoleate vehicle is castor oil.

12. A pharmaceutical formulation according to claim 11 comprising an antioxidant selected from:
0.05% weight of thiourea per volume of formulation; or
0.075% weight of tocopherol per volume of formulation and 2.3% weight of lecithin per volume of formulation.

13. A pharmaceutical formulation as defined in any preceding claim for use in medical therapy.

14. Use of fulvestrant in the preparation of a pharmaceutical formulation as defined in any preceding claim for the treatment of a benign or malignant disease of the breast or reproductive tract.

## Patentansprüche

1. Pharmazeutische Formulierung, enthaltend Fulvestrant, einen Ricinoleat-Hilfsstoff, ein pharmazeutisch annehmbares nichtwässriges Esterlösungsmittel, einen pharmazeutisch annehmbaren Alkohol und ein Antioxidans ausgewählt unter:
Thioharnstoff;
Tocopherol, fakultativ in Gegenwart von Lecithin oder Ascorbylpalmitat;
Ascorbylpalmitat;
Propylgallat, fakultativ in Gegenwart von Butylhydroxyanisol oder Butylhydroxytoluol;
Dithiothreitol;
Butylhydroxytoluol;
Dithioerythreitol;
Acetylcystein;
Thioglycerin; und
Thiomilchsäure,
die zur intramuskulären Anwendung geeignet ist.

2. Formulierung nach Anspruch 1, wobei das Antioxidans unter
Thioharnstoff;
Tocopherol, fakultativ in Gegenwart von Lecithin oder Ascorbylpalmitat;
Ascorbylpalmitat;
Propylgallat, fakultativ in Gegenwart von Butylhydroxyanisol oder Butylhydroxytoluol; und
Dithiothreitol
ausgewählt ist.

3. Formulierung nach Anspruch 1, wobei das Antioxidans unter
Thioharnstoff;
Tocopherol, fakultativ in Gegenwart von Lecithin oder Ascorbylpalmitat;
Propylgallat, fakultativ in Gegenwart von Butylhydroxyanisol; und
Ascorbylpalmitat
ausgewählt ist.

4. Formulierung nach Anspruch 1, wobei das Antioxidans unter
Thioharnstoff; und
Tocopherol, fakultativ in Gegenwart von Lecithin oder Ascorbylpalmitat
ausgewählt ist.

5. Formulierung nach Anspruch 1, wobei das Antioxidans unter
Thioharnstoff; und
Tocopherol, in Gegenwart von Lecithin
ausgewählt ist.

6. Formulierung nach Anspruch 1, wobei es sich bei dem Antioxidans um Thioharnstoff handelt.

7. Pharmazeutische Formulierung nach einem der vorangehenden Ansprüche, wobei es sich bei dem pharmazeutisch annehmbaren Alkohol um ein Gemisch aus Ethanol und Benzylalkohol handelt.

8. Pharmazeutische Formulierung nach einem der vorangehenden Ansprüche, wobei das pharmazeutisch annehmbare nichtwässrige Esterlösungsmittel unter Benzylbenzoat, Ethyloleat, Isopropylmyristat, Isopropylpalmitat oder einem beliebigen Gemisch davon ausgewählt ist.

9. Pharmazeutische Formulierung nach Anspruch 8, wobei es sich bei dem pharmazeutisch annehmbaren nichtwässrigen Esterlösungsmittel um Benzylbenzoat handelt.

10. Dosiseinheit einer pharmazeutischen Formulierung nach einem der Ansprüche 1 bis 9, wobei die Gesamtmenge an Fulvestrant in der Formulierung 250 mg oder mehr und das Gesamtvolumen der Formulierung 6 ml oder weniger ausmachen.

11. Pharmazeutische Formulierung nach einem der vorangehenden Ansprüche, wobei es sich bei dem pharmazeutisch annehmbaren Alkohol um ein Gemisch von, jeweils bezogen auf das Volumen der Formulierung, 10 Gew.-% Ethanol, 10 Gew.-% Benzylalkohol und 15 Gew.-% Benzylbenzoat, sowie bei der Ricinoleat-Trägersubstanz um Rizinusöl handelt.

12. Pharmazeutische Formulierung nach Anspruch 11, enthaltend ein Antioxidans ausgewählt unter:
0,05 Gew.-% Thioharnstoff pro Volumen der Formulierung; oder
0,075 Gew.-% Tocopherol und 2,3 Gew.-% Lecithin, jeweils bezogen auf das Volumen der Formulierung.

13. Pharmazeutische Formulierung gemäß Definition eines der vorangehenden Ansprüche zur Verwendung in der medizinischen Therapie.

14. Verwendung von Fulvestrant bei der Herstellung einer wie in einem der vorangehenden Ansprüche definierten pharmazeutischen Formulierung zur Behandlung gutartiger oder bösartiger Erkrankungen der Brust oder des Reproduktionstrakts.

## Revendications

1. Préparation pharmaceutique adaptée à une administration intramusculaire comprenant du fulvestrant, un excipient de ricinoléate, un solvant d'ester non aqueux acceptable d'un point de vue pharmaceutique, un alcool acceptable d'un point de vue pharmaceutique et un antioxydant choisi parmi :
la thiourée ;
le tocophérol, facultativement en présence de lécithine ou de palmitate d'ascorbyle ;
le palmitate d'ascorbyle ;
le gallate de propyle, facultativement en présence d'hydroxyanisole butylé ou d'hydroxytoluène butylé ;
l'hydroxytoluène ;
le dithiothréitol ;
l'hydroxytoluène butylé ;
le dithioérythréitol ;
l'acétyl cystéine ;
le thioglycérol ; et
l'acide thiolactique.

2. Préparation selon la revendication 1, dans laquelle l'antioxydant est choisi parmi :
la thiourée ;
le tocophérol, facultativement en présence de lécithine ou de palmitate d'ascorbyle ;
le palmitate d'ascorbyle ;
le gallate de propyle, facultativement en présence d'hydroxyanisole butylé ou d'hydroxytoluène butylé ; et
le dithiothréitol.

3. Préparation selon la revendication 1, dans laquelle l'antioxydant est choisi parmi :
la thiourée ;
le tocophérol, facultativement en présence de lécithine ou de palmitate d'ascorbyle ;
le gallate de propyle, facultativement en présence d'hydroxyanisole butylé ; et
le palmitate d'ascorbyle.

4. Préparation selon la revendication 1, dans laquelle l'antioxydant est choisi parmi :
la thiourée ; et
le tocophérol, facultativement en présence de lécithine ou de palmitate d'ascorbyle.

5. Préparation selon la revendication 1, dans laquelle l'antioxydant est choisi parmi :
la thiourée ; et
le tocophérol en présence de lécithine.

6. Préparation selon la revendication 1, dans laquelle l'antioxydant est la thiourée.

7. Préparation pharmaceutique selon l'une quelconque des revendications précédentes, dans laquelle l'alcool, acceptable d'un point de vue pharmaceutique, est un mélange d'éthanol et d'alcool benzylique.

8. Préparation pharmaceutique selon l'une quelconque des revendications précédentes, dans laquelle le solvant d'ester non aqueux, acceptable d'un point de vue pharmaceutique, est choisi parmi le benzoate de benzyle, l'oléate d'éthyle, le myristate d'isopropyle, le palmitate d'isopropyle ou un mélange de n'importe lesquels d'entre eux.

9. Préparation pharmaceutique selon la revendication 8, dans laquelle le solvant d'ester non aqueux, acceptable d'un point de vue pharmaceutique, est le benzoate de benzyle.

10. Dose unitaire d'une préparation pharmaceutique selon l'une quelconque des revendications 1 à 9, dans laquelle la quantité totale de fulvestrant dans la préparation est de 250 mg ou plus et le volume total de préparation est de 6 ml ou moins.

11. Préparation pharmaceutique selon l'une quelconque des revendications précédentes, dans laquelle l'alcool, acceptable d'un point de vue pharmaceutique, est un mélange de 10% en poids d'éthanol par volume de préparation, de 10% en poids d'alcool benzylique par volume de préparation et de 15% en poids de benzoate de benzyle par volume de préparation et le véhicule de ricinoléate est l'huile de ricin.

12. Préparation pharmaceutique, selon la revendication 11, comprenant un antioxydant choisi parmi :
0,05% en poids de thiourée par volume de préparation ; ou
0,075% en poids de tocophérol par volume de préparation et de 2,3% en poids de lécithine par volume de préparation.

13. Préparation pharmaceutique, telle qu'elle est définie dans l'une quelconque des revendications précédentes, destinée à être utilisée dans une thérapie médicale.

14. Utilisation de fulvestrant dans l'élaboration d'une préparation pharmaceutique, telle qu'elle est définie dans l'une quelconque des revendications précédentes, pour le traitement d'une maladie bénigne ou maligne du sein ou de l'appareil reproducteur.
